# EUROPÄISCHE PATENTSCHRIFT

(11) **EP 3 119 521 B1**
(45) Veröffentlichungstag und Bekanntmachung des Hinweises auf die Patenterteilung: **06.03.2019**
(21) Anmeldenummer: 15712302.7
(22) Anmeldetag: 17.03.2015
(51) Int. Cl.: B01L 9/06, C12M 1/24, C12M 3/00

(54) **VORRICHTUNG ZUM AUFNEHMEN UND LAGERN VON BEHÄLTNISSEN**
DEVICE FOR RECEIVING AND STORING CONTAINERS
DISPOSITIF DE RÉCEPTION ET DE RANGEMENT DE CONTENANTS

(30) Priorität: 21.03.2014 DE 102014103930
(43) Veröffentlichungstag der Anmeldung: 25.01.2017
(73) Patentinhaber: Andreas Hettich GmbH & Co. KG, 78532 Tuttlingen (DE)
(72) Erfinder: EBERLE, Klaus-Günter, 78532 Tuttlingen (DE); SCHALDECKER, Natascha, 78532 Tuttlingen (DE)
(74) Vertreter: Puschmann Borchert Bardehle Patentanwälte Partnerschaft mbB
(86) Internationale Anmeldenummer: PCT/EP2015/055588
(87) Internationale Veröffentlichungsnummer: WO 2015/140187

(56) Entgegenhaltungen:
- EP-A1- 1 442 674
- US-A- 2 568 405
- US-A- 3 199 684
- US-A- 4 407 958
- US-A- 5 150 784
- US-A1- 2009 101 539

## Beschreibung

Die Erfindung betrifft eine Vorrichtung zum Aufnehmen und Lagern von Behältnissen, insbesondere von Reagenzgläsern, gemäß der im Oberbegriff des Patentanspruches 1 angegebenen Art.

Zur Kultivierung bestimmter Mikroorganismen werden feste Nährmedien eingesetzt, bei denen in der Regel Agar-Agar (auch: Agar) als verfestigender Bestandteil des Nährmediums verwendet wird. Die Zubereitung des Nährmediums erfolgt durch Vermischen des in Pulverform vorliegenden Agars sowie ggf. weiterer Bestandteile mit heißem Wasser. Beim Abkühlen erhärtet sich das Nährmedium.

Während zur Kultivierung von Mikroorganismen auch Petrischalen verwendet werden, werden mitunter Reagenzgläser vorgezogen, unter anderem da sie platzsparender gelagert und einfacher verschlossen werden können und da das Risiko des Verschüttens geringer ist. Allerdings ist die Oberfläche des Mediums in senkrecht positionierten Reagenzgläsern naturgemäß klein. Um eine größere Oberfläche zu erzielen, ist es daher üblich, die Reagenzgläser schräg anzustellen, insbesondere während der Abkühl- und Aushärtephase. Man nennt auf diese Weise hergestellte Nährmedien auch Schrägagar. Je nach Anwendung werden dabei unterschiedliche Winkel bevorzugt, häufig 5° oder 20° zur Aufstandsfläche, wobei sich der Winkel auf die Längsachse des Reagenzglases, in das der Schrägagar eingebracht und gelagert ist, zur Aufstandsfläche des Reagenzglases bezieht, also auf die durch die Auflager definierte Lagerachse, welche die Ausrichtung des eingebrachten Behältnisses bestimmt. Bei einem zylindrischen Behältnis sind die Lagerachse und die Längsachse des Behältnisses parallel.

Aus der US 3 199 684 ist ein Gestell für Reagenzgläser bekannt. Dieses weist ein Gehäuse, mit in das Gehäuse eingebrachte gewellte Auflagefächer auf. Die Innenseite des Gehäuses bildet eine Aufstandsfläche. Zudem ist ein Klappmechanismus vorgesehen, der mit dem Gehäuse und den Auflagefächern verbunden ist. Der Klappmechanismus richte die Auflagefächer in verschiedenen Klappstellungen in verschiedene Winkel zur Aufstandsfläche aus. Das Gestell weist zudem einen Winkelmesser auf. Die Auflagefächer und Aufstandsfläche sind parallel zu einer Längsachse der Vorrichtung ausgerichtet. Die Auflagefächer sind identisch dimensioniert und U-förmig und nach oben offen ausgebildet.

Aus der US 2009/0101539 A1 ist ein nach oben offener Behälter zum Aufnehmen und Lagern von Behältnissen bekannt. Der Behälter umfasst dabei ein Gehäuse, in das eine Halteleiste mit Ausnehmungen eingebracht ist. Auf dem Boden innerhalb des Gehäuses sind Aufnahmen für die Behältnisse vorgesehen. Zwei Griffe bildende Bügel sind mit dem Gehäuse verbunden. Durch seitliches Abklappen der Bügel, in denen die Bügel fixiert werden, kann der Behälter in unterschiedlichen Winkeln zur Aufstandsfläche schräg ausgerichtet werden. Im geschlossenen Zustand der Bügel, dienen diese als Anschlagwand, deren Länge dem Boden des Gehäuses entspricht.

Aus der EP 1 442 674 A1 ist ein einstellbares Weinregal zum Aufnehmen und Lagern von Flaschen offenbart. Das Weinregal umfasst einen Rahmen und ein in dem Rahmen ausklappbares Gestell mit Quer- und Längsstäben. Das Gestell ist in verschiedenen Klappstellungen mit verschiedenen Winkeln zur Aufstandsfläche bewegbar.

In der US 4,407,958 wird ein Gestell vorgeschlagen, in dem Reagenzgläser sowohl vertikal als auch in den oben erwähnten Winkeln von 5° und 20° gelagert werden können. Das Gestell besteht im Wesentlichen aus einer rechteckigen Tragplatte, zwei parallel darüber angeordneten Halteplatten selben Ausmaßes und zwei Verbindungsplatten, durch weiche die Trag- und Halteplatten jeweils an ihren Breitseiten voneinander sowie übereinander beabstandet und über seitliche Verbindungsplatten miteinander verbunden sind. Die Tragplatte und die Halteplatten weisen eine Vielzahl von Ausnehmungen auf, die einander so zugeordnet und so bemessen sind, dass ein Reagenzglas mit seinem unteren Ende durch eine erste Ausnehmung in der oberen Halteplatte und eine zugeordnete zweite Ausnehmung in der zweiten Halteplatte geführt wird und schließlich das untere Ende des Reagenzglases von einer gegenüber dem Reagenzglas kleiner bemessenen dritten Ausnehmung in der Tragplatte aufgenommen und axial fixiert wird. Die Verbindungsplatten sind jeweils in zwei Plattenbereiche gegliedert, welche miteinander über einen Steg verbunden sind und eine in etwa v-förmige, nach unten offene Ausnehmung bilden. Die beiden Plattenbereiche weisen jeweils eine innere Kante auf, die aufeinander gerichtet sind und schräg aufeinander zulaufen. Die Unterkanten der beiden Plattenbereiche sind parallel zur Aufstandsfläche und parallel zu den Trag- und Halteplatten ausgerichtet und bilden jeweils einen Fuß für die Aufstandsfläche des Gestells. Die Verbindungsplatten verlaufen senkrecht zu den Halte- und Tragplatten. Die Außenkanten der Verbindungsplatten sind zur vertikalen Erstreckung des Gestells um 5° bzw. um 20° geneigt, so dass sich die Verbindungsplatten von unten nach oben verbreitern, und zwar auf der ersten Längsseite des Gestells um 5° und auf der zweiten Längsseite um 20°.

Zur Lagerung der Reagenzgläser in einer vertikalen Ausrichtung steht das Gestell auf den Unterkanten der Verbindungsplatten. Zur schrägen Lagerung wird das Gestell auf eine seiner beiden Längsseiten gekippt, so dass es auf den Außenkanten der Verbindungsplatten steht. Durch die Wahl der Längsseite wird somit der Winkel der Ausrichtung der Reagenzgläser bestimmt.

Die zuverlässige Ausrichtung und Lagerung in exakt dem gewünschten Winkel und die relativ unkomplizierte Wahl des Winkels sind zwar vorteilhaft. Der Nachteil dieser Lösung liegt aber zum einen in der Größe des Gestells. In der Ausgangsposition zur vertikalen Ausrichtung der Reagenzgläser betrachtet ist die Höhe der Konstruktion durch die Länge der Reagenzgläser vorgegeben, und für die Stabilität der Konstruktion ist eine gewisse Mindestbreite erforderlich. Dies ist besonders bei Einsatz des Gestells in kleineren Inkubatoren ungünstig. Auch gestaltet sich die Reinigung unter Umständen als problematisch, da das Gestell für kleine Autoklaven zu groß sein kann und auch für die manuelle Reinigung nicht an allen Stellen gut erreichbar ist. Zum anderen erweist sich die Handhabung des Gestells als schwierig, auch auf Grund seiner Größe, da zur Veränderung des Winkels der Ausrichtung das Gestell vollkommen neu ausgerichtet werden muss. Dies ist insbesondere unter beengten Verhältnissen, wie in einem Inkubator, recht aufwendig.

Aufgabe der Erfindung ist es, eine Vorrichtung zum Einsetzen von Behältnissen, insbesondere Reagenzgläsern, zu schaffen, die unter Vermeidung der genannten Nachteile platzsparend ausgeführt ist und leicht gereinigt werden kann.

Diese Aufgabe wird durch die kennzeichnenden Merkmale des Patentanspruches 1 in Verbindung mit seinen Oberbegriffsmerkmalen gelöst.

Der Erfindung liegt die Erkenntnis zugrunde, dass für die schräge Ausrichtung von Behältnissen, insbesondere Reagenzgläsern, nur eine geringe Drehbewegung des Gestells um den gewünschten Winkel ausreichend ist und dass diese Drehbewegung durch einen Einstellmechanismus gewährleistet werden kann. Ein Einstellmechanismus für den Winkel der Ausrichtung kann durch einen Klappmechanismus auf einfache Weise gebildet werden.

Nach der Erfindung weist die Vorrichtung zum Aufnehmen und Lagern von Behältnissen, insbesondere Reagenzgläsern, zumindest eine Auflageleiste mit Ausnehmungen auf, wobei jedem Behältnis eine Ausnehmung in der Auflageleiste zugeordnet ist, die als Auflager dient. Ferner sind eine Vorderseite und eine der Vorderseite gegenüberliegend angeordnete Aufstandsleiste vorgesehen. Die Aufstandsleiste dient der Anlage des Bodens des Behältnisses und der axialen Festlegung des Behältnisses. Der Abstand zwischen der Auflageleiste und der Aufstandsleiste ist so bemessen, dass Reagenzgläser aller marktüblichen Längen stabil in der Vorrichtung gelagert werden können.

Erfindungsgemäß ist eine Bodenplatte vorgesehen, die Auflageleiste und die Aufstandsleiste sind über die Bodenplatte miteinander verbunden, wobei die Auflageleiste senkrecht zur Bodenplatte ausgerichtet ist. Zwischen der ersten Auflageleiste und der Aufstandsleiste ist eine zweite Auflagerleiste angeordnet, welche ein zweites Auflager aufweist. Dabei ist ein Klappmechanismus für zumindest zwei Klappstellungen vorgesehen und mit dem Gehäuse verbunden, der die auf einer Aufstandsfläche aufliegende Vorrichtung in eine erste Klappstellung in einem ersten Winkel zur Aufstandsfläche und in eine zweite Klappstellung in einem zweiten Winkel zur Aufstandsfläche ausrichtet. So kann ohne großen Aufwand unter den im jeweiligen Einsatzgebiet am häufigsten verwendeten Winkeln für die Lagerung von Mikroorganismen der passendste ausgewählt und die Vorrichtung in diesem Winkel ausgerichtet werden. Der Winkel bezieht sich dabei auf die Längsachse des in die Vorrichtung eingebrachten und dort gelagerten Reagenzglases zur Aufstandsfläche der Vorrichtung bezieht und somit auf die Ausrichtung des Auflagers und der Anlage für das Behältnis, Dabei sind die Aufstandsleisten leicht herzustellen. Der Lagerpunkt der Behältnisse auf der zweiten Auflageleiste ist vom Behälterboden beabstandet, was die Lagerung noch sicherer macht.

Gemäß einem Aspekt der Erfindung sind die Auflager zueinander beabstandet angeordnet und das Behältnis in einen dritten Winkel relativ zur Bodenplatte ausgerichtet. Durch die zwei voneinander beabstandeten Auflager wird eine sicherere Lagerung bewirkt. Zudem kann der dritte Winkel so gewählt sein, dass er bereits einem im jeweiligen Einsatzgebiet häufig verwendeten Winkel zur Lagerung entspricht, so dass nach dem Auflegen des Behältnisses auf die Auflager häufig keine weitere Einstellung des Winkels erforderlich ist.

Gemäß einer Ausführungsform der Erfindung hat es sich als vorteilhaft erwiesen, wenn der erste Winkel 5° und der zweite Winkel 20° betragen. In diesem Fall lagern die Behältnisse parallel zur Bodenplatte, also die Längsachse der Behältnisse ist parallel zur Bodenplatte, und über den Klappmechanismus wird einer der beiden Winkel 5° und 20° zur Aufstandsfläche gewählt, d.h. die Auflager für die Behältnisse werden in der Vorrichtung durch den Klappmechanismus so ausgerichtet, dass die Längsachse der in die Vorrichtung eingebrachten Behältnisse mit der Aufstandsfläche der Vorrichtung die genannten Winkel einnehmen kann. Da gewöhnlich Nährmedien mit aeroben Kulturen in einem Winkel von 5° und Nährmedien mit anaeroben Kulturen in einem Winkel von 20° gelagert werden, entspricht diese Wahlmöglichkeit Anforderungen, die in der Praxis häufig anzutreffen sind.

Bei einer vorteilhaften Weiterbildung der Erfindung sind die Auflage- und Aufstandsleisten parallel zu einer Längsachse der Vorrichtung angeordnet. Dies erleichtert die Herstellung der Vorrichtung und dient einer sicheren Lagerung der Behältnisse.

Es ist vorteilhaft, wenn die Auflager u-förmig, nach oben offen und an das Behältnis angepasst ausgebildet sind. Insbesondere ist es dabei günstig, wenn der Querschnitt des Auflagers sich von unten nach oben graduell so vergrößert, dass Reagenzgläser aller marktüblichen Durchmesser stabil in der Vorrichtung gelagert werden können. Dadurch wird die Aufnahme der Behältnisse erleichtert, und die Lagerung wird noch sicherer.

Um den Klappmechanismus möglichst einfach zu bedienen und stabil zu gestalten, weist er eine Anschlagsleiste, deren Länge der Länge der Bodenplatte entspricht, und zwei Befestigungslaschen auf, die an beiden Enden der Anschlagsleiste lotrecht angebracht sind. Die Anschlagsleiste ist dabei über die Befestigungslaschen an den beiden Stirnseiten der Vorrichtung so befestigt, dass sie über eine zur Längsachse parallele Drehachse klappbar ist. Der Lagerungswinkel kann so ganz einfach durch die Position der Anschlagsleiste bestimmt werden. Da sich die Länge der Anschlagsleiste über die gesamte Länge der Bodenplatte erstreckt, ist der Klappmechanismus äußerst stabil.

In einer alternativen Ausführungsform weist die Anschlagsleiste zumindest zwei Füße auf, die insbesondere mit der Anschlagsleiste einstückig und materialeinheitlich ausgebildet sind, und in der ersten Klappstellung die Füße und die Anschlagleiste eine ebene Auflagefläche bilden. Dadurch kann die Anschlagleiste schmaler ausgeführt werden, wodurch Material eingespart und Gewicht verringert wird. Bei günstiger Positionierung der Füße, insbesondere an den beiden Enden der Anschlagleiste, bleibt die Stabilität der Konstruktion unverändert hoch.

Vorzugsweise sind die Befestigungslaschen jeweils an einem Wandstück über ein Drehgelenk befestigt, welches an beiden Stirnseiten vorgesehen und senkrecht zur Bodenplatte ausgerichtet angeordnet ist. So lässt sich eine einfache und stabile Befestigung des Klappmechanismus an der Vorrichtung mit geringem Material- und Konstruktionsaufwand realisieren.

Weiter hat es sich als vorteilhaft erwiesen, dass die Anschlagsleiste in der ersten Klappstellung mit den Füßen an der Unterseite der Bodenplatte anliegt und dass die Anschlagsleiste in der zweiten Klappstellung an einer Auflageleiste anliegt und die Füße auf der Aufstandsfläche aufstehen. So werden der erste Winkel und der zweite Winkel durch die Ausbildung der Anschlagleiste, im Wesentlichen ihrer Dicke und Höhe, bestimmt, wobei durch das Anliegen der Anschlagleiste, entweder an der Bodenplatte oder an einer Auflageleiste, über ihre gesamte Länge eine optimale Stabilität gewährleistet ist.

Es ist besonders vorteilhaft, wenn die Auflageleisten identisch dimensioniert sind. Dies vereinfacht sowohl die Herstellung als auch das Zusammensetzen der Vorrichtung, beispielsweise nach einer Reinigung.

Bei einer vorteilhaften Weiterbildung der Erfindung sind an den Stirnseiten Griffe angeordnet. Dies erleichtert den Transport der Vorrichtung.

In einer weiteren vorteilhaften Ausgestaltung sind die Griffe in einem solchen Abstand zur Bodenplatte angeordnet, dass diese sich oberhalb der Auflageleiste befinden, insbesondere in einen Abstand, der größer ist als der Radius der u-förmigen Ausnehmung, welche das Auflager bildet. Dies macht zum einen die Handhabung und den Transport der Vorrichtung sicherer, da der Schwerpunkt der Vorrichtung unterhalb der Griffe liegt. Zum anderen können die Griffe leichter so ausgeführt werden, dass auf ihnen baugleiche Vorrichtungen einrasten und dort gelagert werden können.

Gemäß einem Aspekt der Erfindung ist die Vorrichtung autoklavierbar ausgebildet, insbesondere durch eine Herstellung aus Edelstahl. So ist die Vorrichtung kratz- und abriebfest, durch Säuren und Laugen nicht angreifbar, rostfrei und temperaturbeständig und kann effizient gereinigt werden.

Weitere Vorteile, Merkmale und Anwendungsmöglichkeiten der vorliegenden Erfindung ergeben sich aus der nachfolgenden Beschreibung in Verbindung mit den in den Zeichnungen dargestellten Ausführungsbeispielen.

In der Beschreibung, in den Ansprüchen und in der Zeichnung werden die in der unten aufgeführten Liste der Bezugszeichen verwendeten Begriffe und zugeordneten Bezugszeichen verwendet. In der Zeichnung bedeutet:
- Fig. 1: eine perspektivische Draufsicht auf eine Ausführungsform einer Vorrichtung zum Aufnehmen und Lagern von Behältnissen nach der Erfindung;
- Fig. 2: eine Seitenansicht der Ausführungsform von Fig. 1, und
- Fig. 3: eine Frontansicht der Ausführungsform von Fig. 1.

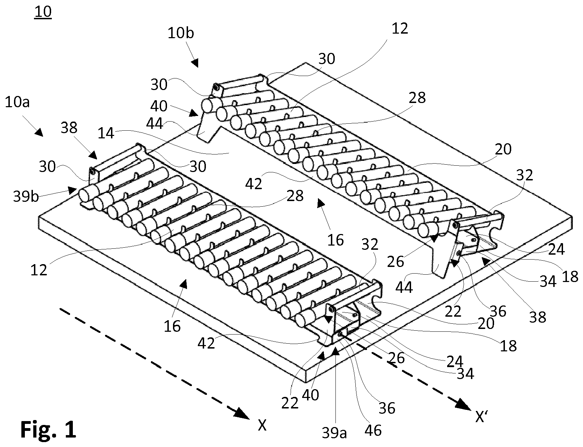

In Fig. 1 wird eine perspektivische Draufsicht auf zwei erfindungsgemäße parallel zueinander aufgestellte Lagervorrichtungen 10 mit darin gelagerten Reagenzgläsern 12 gezeigt. Der Unterschied besteht lediglich darin, dass durch die jeweils gewählte Position des Klappmechanismus 40 die vordere Lagervorrichtung 10a in einem kleineren Winkel zur Aufstandsfläche 14 angestellt ist als die dahinterliegende Lagervorrichtung 10b, d.h. die Auflager für die Reagenzgläser 12 sind in der Lagervorrichtung 10 so ausgerichtet, dass die Längsachse der in die Lagervorrichtung 10 eingebrachten Reagenzgläser 12 mit der Aufstandsfläche 14 der Lagervorrichtung 10 die genannten Winkel einnehmen kann.

Beide Lagervorrichtungen 10 weisen jeweils eine Vorderseite 16 und eine Bodenplatte 18 auf, auf der lotrecht an der Vorderseite 16 eine erste Auflageleiste 22, eine der Vorderseite 16 gegenüberliegende Aufstandsleiste 20 und eine zwischen der ersten Auflageleiste 22 und der Aufstandsleiste 20 liegende zweite Auflageleiste 24 angeordnet sind. Die lagernden Reagenzgläser 12 liegen mit ihren Böden an der Aufstandsleiste 20 an und werden durch diese axial festgelegt. Des Weiteren sind den Reagenzgläsern 12 Ausnehmungen 26 in der ersten Auflageleiste 22 und Ausnehmungen 28 in der zweiten Auflageleiste 24 zugeordnet. Die Ausnehmungen 26 und 28 sind u-förmig und nach oben offen ausgebildet und legen die Reagenzgläser 12 bezüglich einer Längsachse x der Lagervorrichtung 10 fest. Sowohl die Aufstandsleiste 20 als auch die erste Auflageleiste 22 bilden jeweils an beiden Enden 39a und 39b Erhöhungen 30 aus, die in der Höhe über den Radius der Ausnehmungen 26 und 28 hinausragen. Jeweils die beiden entlang der Längsachse x einander gegenüberliegenden Erhöhungen 30 sind durch eine Strebe verbunden, die als Griff 32 dient.

An beiden Stirnseiten 38 der Lagervorrichtungen 10 sind lotrecht zur Bodenplatte 18 zwischen der ersten Auflageleiste 22 und der zweiten Auflageleiste 24 Wandstücke 34 vorgesehen, die jeweils ein Drehgelenk 36 aufweisen, an dem ein Klappmechanismus 40 um eine zur Längsachse x parallele Drehachse x' drehbar befestigt ist. Der Klappmechanismus 40 weist eine Anschlagleiste 42 auf mit derselben Länge wie die Bodenplatte 18 und mit zwei einstückig an ihren Enden 39a und 39b ausgebildeten Füßen 44 sowie zwei Befestigungslaschen 46. Die Befestigungslaschen 46 sind jeweils lotrecht zu stirnseitigen Enden 39a, 39b der Anschlagleiste 42 angeordnet und mit den Drehgelenken 36 fest verbunden.

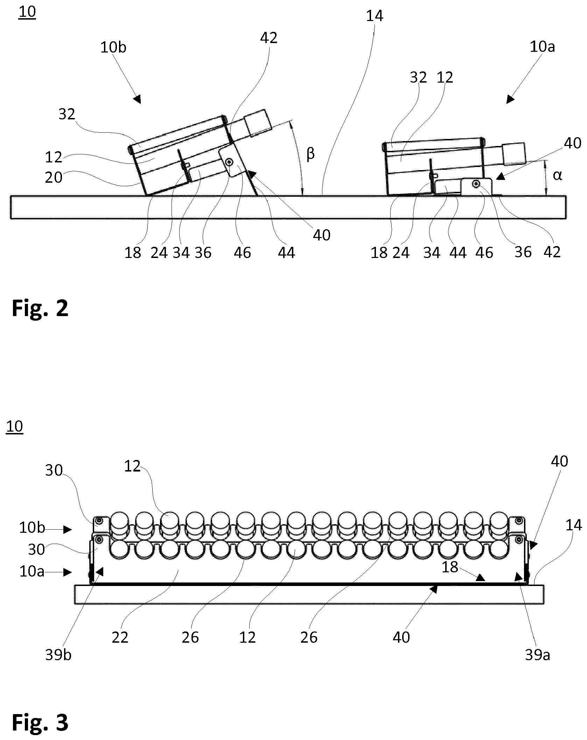

Bei der Lagervorrichtung 10a ist die Anschlagleiste 42 unter die Bodenplatte 18 eingeklappt, wodurch die Reagenzgläser 12 in einem ersten Winkel α zur Aufstandsfläche 14 angestellt sind. Bei der Lagervorrichtung 10b ist die Anschlagleiste 42 ausgeklappt, und ihre Füße 44 stehen auf der Aufstandsfläche 14. Daraus resultiert ein größerer zweiter Winkel β die Reagenzgläser 12 zur Aufstandsfläche 14 angestellt sind. Der besseren Übersichtlichkeit wegen sind die Winkel α und β in Fig. 2 eingezeichnet.

In Fig. 2 sind die Lagervorrichtungen 10a und 10b aus Fig. 1 in Seitenansicht dargestellt. Die im Wesentlichen zylindrischen Reagenzgläser 12 lagern, wie zuvor beschrieben, in auf der ersten Auflageleiste 22 vorgesehenen Ausnehmungen 26 und in auf der zweiten Auflageleiste 24 vorgesehenen Ausnehmungen 28. Sind die Reagenzgläser 12 in die Lagervorrichtung 10a und 10b eingebracht, werden die Ausnehmungen 26 und 28 durch das eingebrachte Reagenzglas 12 jeweils miteinander verbunden. Eine Lagerachse L der beiden Ausnehmungen 26 und 28 ist dabei parallel zu der Längsachse des eingebrachten Reagenzglases 12. Die Lagerachse L der beiden Ausnehmungen, aber auch die Längsachse des eingebrachten Reagenzglases 12 bildet mit der Aufstandsfläche jeweils die oben und unten genannten Winkel.

In der Lagervorrichtung 10a ist der Klappmechanismus 40 über mit seinen Befestigungslaschen 46 verbundene Drehgelenke 36 unter die Bodenplatte 18 eingeklappt, so dass die Füße 44 und die Anschlagleiste 42 an der Aufstandsfläche 14 anliegen. Die Maße der Befestigungslaschen 46 und der Abstand der Drehgelenke 36 von der Bodenplatte 16 sind dabei so bemessen, dass die Reagenzgläser 12 in einem ersten Winkel α von 5° zur Aufstandsfläche 14 angestellt sind.

In der Lagervorrichtung 10b ist der Klappmechanismus 40 ausgeklappt, so dass die Füße 44 auf der Aufstandsfläche 14 aufstehen und die Anschlagleiste 42 an der ersten Auflageleiste 22 anliegt. In dieser Klappstellung des Klappmechanismus 40 sind die Reagenzgläser 12 in einem zweiten Winkel β von 20° zur Aufstandsfläche 14 angestellt.

Die Winkel α und β können entweder, wenn die Abstände der tiefsten Punkte der Ausnehmungen 26 und der Ausnehmungen 28 von der Bodenplatte 18 gleich sind und die Reagenzgläser 12 somit parallel zur Bodenplatte 18 gelagert sind, allein durch die Maße und die Anordnung des Klappmechanismus 40 festgelegt werden. Oder die Reagenzgläser 12 können durch verschiedene Abstände der tiefsten Punkte der Ausnehmungen 26 und der Ausnehmungen 28 von der Bodenplatte 18 in einem bestimmten Winkel zur Bodenplatte 18 angestellt sein, der dann bei der Bestimmung der Maße und der Anordnung des Klappmechanismus 40 mit einbezogen wird.

In Fig. 3 sind die Lagervorrichtungen 10 in Frontansicht dargestellt. Aus dieser Perspektive ist der Unterschied zwischen dem Winkel α, in dem die Reagenzgläser 12 in der Lagervorrichtung 10a gelagert sind, und dem Winkel β, in dem die Reagenzgläser 12 in der Lagervorrichtung 10b gelagert sind, gut zu erkennen. In der Lagervorrichtung 10a ist der Klappmechanismus 40 unter die Bodenplatte 18 eingeklappt, und an der Vorderseite 16 ist die erste Auflageleiste 22 vollständig sichtbar. Die Reagenzgläser 12 lagern in Ausnehmungen 26. An beiden Enden 39a und 39b der ersten Auflageleiste 22 sind Erhöhungen 30 ausgebildet, die durch aus dieser Perspektive nicht ersichtliche Griffen 32 mit ebenfalls nicht ersichtlichen Enden 39a und 39b der Aufstandsleiste 20 verbunden sind. Allerdings ist auf Grund des größeren Winkels β in der Lagervorrichtung 10b erkennbar, wie die Reagenzgläser 12 dort ebenfalls in den Ausnehmungen 26 gelagert sind.

### Bezugszeichenliste

- 10: Lagervorrichtung
- 12: Reagenzgläser
- 14: Aufstandsfläche
- 16: Vorderseite
- 18: Bodenplatte
- 20: Aufstandsleiste
- 22: erste Auflageleiste
- 24: zweite Auflageleiste
- 26: Ausnehmungen
- 28: Ausnehmungen
- 30: Erhöhungen
- 32: Griffe
- 34: Wandstücke
- 36: Drehgelenke
- 38: Stirnseiten
- 39: Enden
- 40: Klappmechanismus
- 42: Anschlagleiste
- 44: Füße
- 46: Befestigungslaschen

- α: erster Winkel
- β: zweiter Winkel

## Patentansprüche

1. Vorrichtung (10) zum Aufnehmen und Lagern von Behältnissen (12) mit einem Gehäuse umfassend:
- zumindest eine Auflageleiste (22) mit Ausnehmungen (26), wobei jedem Behältnis (12) eine Ausnehmung (26) in der Auflageleiste (22) zugeordnet ist, die als erstes Auflager (26) dient,
- eine Vorderseite (16),
- eine der Vorderseite (16) gegenüberliegend angeordnete Aufstandsleiste (20) zur Anlage des Bodens des Behältnisses (12) und zur axialen Festlegung des Behältnisses (12),
wobei eine Bodenplatte (18) vorgesehen ist, die Auflageleiste (22) und die Aufstandsleiste (20) über die Bodenplatte (18) miteinander verbunden sind, wobei die Auflageleiste (22) senkrecht zur Bodenplatte (18) ausgerichtet ist, wobei zwischen der ersten Auflageleiste (22) und der Aufstandsleiste (20) eine zweite Auflagerleiste (24) angeordnet ist, welche ein zweites Auflager (28) aufweist, und ein Klappmechanismus (40) für zumindest zwei Klappstellungen vorgesehen und mit dem Gehäuse verbunden ist, der die auf einer Aufstandsfläche (14) aufliegende Vorrichtung in eine erste Klappstellung in einem ersten Winkel (α) zur Aufstandsfläche (14) und in eine zweite Klappstellung in einem zweiten Winkel (β) zur Aufstandsfläche (14) ausrichtet, wobei sich der erste und der zweite Winkel auf eine Lagerachse (L) für das Behältnis (12) und der Aufstandsfläche (14) bezieht.

2. Vorrichtung nach Anspruch 1, **dadurch gekennzeichnet, dass** die Auflager (26, 28) insbesondere zueinander beabstandet angeordnet sind und das Behältnis (12) in einen dritten Winkel relativ zur Bodenplatte (18) ausrichten.

3. Vorrichtung nach Anspruch 1 oder 2, **dadurch gekennzeichnet, dass** der erste Winkel 5° und der zweite Winkel 20° beträgt.

4. Vorrichtung nach einem der vorangehenden Ansprüche, **dadurch gekennzeichnet, dass** die Auflageleisten (22, 24) und die Aufstandsleiste (20) parallel zu einer Längsachse (x) der Vorrichtung angeordnet sind.

5. Vorrichtung nach einem der vorangehenden Ansprüche, **dadurch gekennzeichnet, dass** die Auflager (26,28) u-förmig, nach oben offen und an das Behältnis (12) angepasst ausgebildet sind,

6. Vorrichtung nach einem der vorangehenden Ansprüche, **dadurch gekennzeichnet, dass** der Klappmechanismus (40) eine Anschlagsleiste (42), deren Länge der Länge der Bodenplatte (18) entspricht, und zwei Befestigungslaschen (46) aufweist, die an beiden Enden (39a, b) der Anschlagsleiste (42) lotrecht angebracht sind, wobei die Anschlagsleiste (42) über die Befestigungslaschen (46) an den beiden Stirnseiten (38) der Vorrichtung so befestigt ist, dass sie über eine zur Längsachse (x) parallele Drehachse (x') klappbar ist.

7. Vorrichtung nach Anspruch 6, **dadurch gekennzeichnet, dass** die Anschlagsleiste (42) zumindest zwei Füße (44) aufweist, die insbesondere mit der Anschlagsleiste (42) einstückig und materialeinheitlich ausgebildet sind, und in der ersten Klappstellung die Füße (44) und die Anschlagleiste eine ebene Auflagefläche bilden.

8. Vorrichtung nach Anspruch 7, **dadurch gekennzeichnet, dass** die Befestigungslaschen (46) jeweils an einem Wandstück über ein Drehgelenk befestigt sind, welches an beiden Stirnseiten vorgesehen und senkrecht zur Bodenplatte (18) ausgerichtet angeordnet ist.

9. Vorrichtung nach Anspruch 7 oder 8, **dadurch gekennzeichnet, dass** die Anschlagsleiste (42) in der ersten Klappstellung mit den Füßen (44) an der Unterseite der Bodenplatte (18) anliegt und dass die Anschlagsleiste (42) in der zweiten Klappstellung an der ersten Auflageleiste (22) anliegt und die Füße (44) auf der Aufstandsfläche (14) aufstehen.

10. Vorrichtung nach einem der vorangehenden Ansprüche, **dadurch gekennzeichnet, dass** die Auflageleisten (22,24) identisch dimensioniert sind.

11. Vorrichtung nach einem der vorangehenden Ansprüche, **dadurch gekennzeichnet, dass** an den Stirnseiten (38) Griffe (32) angeordnet sind.

12. Vorrichtung nach Anspruch 11, **dadurch gekennzeichnet, dass** die Griffe (32) in einem solchen Abstand zur Bodenplatte (18) angeordnet sind, dass diese sich oberhalb der Auflageleisten (22, 24) befinden, insbesondere in einen Abstand oberhalb der Auflageleisten (22, 24), der größer ist als der Radius der u-förmigen Ausnehmungen (26, 28), welche das Auflager bilden,

13. Vorrichtung nach einem der vorangehenden Ansprüche, **gekennzeichnet durch** eine autoklavierbare Ausbildung, insbesondere **durch** eine Ausbildung aus Edelstahl.

## Claims

1. Device (10) for receiving and storing containers (12) with a housing, comprising:
- at least one bearing strip (22) with recesses (26), with every container (12) being assigned one recess (26) each in the bearing strip (22) that serves as a first support (26),
- a front (16),
- an abutment strip (20) arranged opposite said front (16), on which the bottom of the container (12) abuts and which axially secures said container (12),
wherein a base plate (18) is provided, said bearing strip (22) and said abutment strip (20) are connected to one another via said base plate (18), wherein said abutment strip (22) is aligned perpendicularly to said base plate (18), wherein a second bearing strip (24) having a second support (28) is arranged between said first bearing strip (22) and said abutment strip (20), and a folding mechanism (40) for at least two folding positions is provided and connected to said housing, which mechanism moves the device abutting on a contact surface (14) into a first folding position, at a first angle (α) relative to the contact surface (14), and into a second folding position, at a second angle (β) relative to the contact surface (14), with said first and second angles relating to a bearing axis (L) for the container (12) and the contact surface (14).

2. Device according to claim 1, **characterized in that** said supports (26, 28) are arranged in particular at a distance from one another and align the container (12) at a third angle relative to the base plate (18).

3. Device according to claim 1 or claim 2, **characterized in that** said first angle is 5° and that said second angle is 20°.

4. Device according to any one of the preceding claims, **characterized in that** said bearing strips (22, 24) and said abutment strip (20) are arranged parallel to a longitudinal axis (x) of the device.

5. Device according to any one of the preceding claims, **characterized in that** said supports (26, 28) are U-shaped, open at the top and are designed to fit the container (12).

6. Device according to any one of the preceding claims, **characterized in that** said folding mechanism (40) has a stop strip (42), the length of which corresponds to the length of the base plate (18), and includes two mounting lugs (46) which are mounted vertically at both ends (39a, b) of the stop strip (42), said stop strip (42) being mounted, via the mounting lugs (46), to the two end faces (38) of the device in such a way that it can be folded over an axis of rotation (x') parallel to the longitudinal axis (x).

7. Device according to claim 6, **characterized in that** the stop strip (42) has at least two feet (44), which in particular are integrally formed with, and made of the same material as, the stop strip (42), and, in the first folding position, the feet (44) and the stop rail form a flat bearing surface.

8. Device according to claim 7, **characterized in that** the mounting lugs (46) are each mounted on a wall section via a swivel joint which is provided on both end faces and arranged perpendicularly to the base plate (18).

9. Device according to claim 7 or claim 8, **characterized in that,** in the first folding position, the stop strip (42) has its feet (44) resting on the underside of the base plate (18) and, in the second folding position, the stop strip (42) rests on the first bearing strip (22) and the feet (44) rest on the contact surface (14).

10. Device according to any one of the preceding claims, **characterized in that** said bearing strips (22, 24) are identically dimensioned.

11. Device according to any one of the preceding claims, **characterized in that** handles (32) are arranged on the end faces (38).

12. Device according to claim 11, **characterized in that** said handles (32) are arranged at such a distance from the base plate (18) that they are located above the bearing strips (22, 24), in particular at a distance above the bearing strips (22, 24) which is larger than the radius of the U-shaped recesses (26, 28) which form the support.

13. Device according to any one of the preceding claims, **characterized by** an autoclavable design, in particular by a design made of stainless steel.

## Revendications

1. Dispositif (10) de logement et de support de récipients (12) comprenant un boîtier, comportant :
- au moins une barre d'appui (22) pourvue d'évidements (26), dans lequel un évidement (26) dans la barre d'appui (22), lequel sert de premier appui (26), est associé à chaque récipient (12),
- une face avant (16),
- une barre de support (20) agencée à l'opposé de la face avant (16) destinée à venir en contact avec le fond du récipient (12) et à immobiliser axialement le récipient (12),
dans lequel une plaque de fond (18) est prévue, la barre d'appui (22) et la barre de support (20) sont reliées l'une à l'autre par la plaque de fond (18), dans lequel la barre d'appui (22) est orientée perpendiculairement à la plaque de fond (18), dans lequel une deuxième barre d'appui (24) est agencée entre la première barre d'appui (22) et la barre de support (20) et présente un deuxième appui (28), et
un mécanisme de basculement (40) pour au moins deux positions de basculement est prévu et relié au boîtier, lequel mécanisme oriente le dispositif reposant sur une surface de support (14) dans une première position de basculement en formant un premier angle (α) par rapport à la surface de support (14) et dans une deuxième position de basculement en formant un deuxième angle (β) par rapport à la surface de support (14), dans lequel le premier et le deuxième angle se rapportent à un axe de support (L) pour le récipient (12) et la surface de support (14).

2. Dispositif selon la revendication 1, **caractérisé en ce que** les appuis (26, 28) sont agencés en particulier à une certaine distance l'un de l'autre et orientent le récipient (12) en formant un troisième angle par rapport à la plaque de fond (18).

3. Dispositif selon la revendication 1 ou 2, **caractérisé en ce que** le premier angle atteint 5° et le deuxième angle atteint 20°.

4. Dispositif selon l'une quelconque des revendications précédentes, **caractérisé en ce que** les barres d'appui (22, 24) et la barre de support (20) sont agencées parallèlement à un axe longitudinal (x) du dispositif.

5. Dispositif selon l'une quelconque des revendications précédentes, **caractérisé en ce que** les appuis (26, 28) sont ouverts vers le haut en forme de u et sont adaptés au récipient (12).

6. Dispositif selon l'une quelconque des revendications précédentes, **caractérisé en ce que** le mécanisme de basculement (40) présente une barre de butée (42), dont la longueur correspond à la longueur de la plaque de fond (18), et deux languettes de fixation (46), qui sont montées verticalement aux deux extrémités (39a, b) de la barre de butée (42), dans lequel la barre de butée (42) est fixée aux deux faces frontales (38) du dispositif par l'intermédiaire des languettes de fixation (46), de manière à pouvoir basculer par l'intermédiaire d'un axe de rotation (x') parallèle à l'axe longitudinal (x).

7. Dispositif selon la revendication 6, **caractérisé en ce que** la barre de butée (42) comprend au moins deux pieds (44), qui sont réalisés d'une seule pièce avec la barre de butée (42) avec homogénéité de matériau, et, dans la première position de basculement, les pieds (44) et la barre de butée forment une surface d'appui plate.

8. Dispositif selon la revendication 7, **caractérisé en ce que** les languettes de fixation (46) sont fixées chacune à une pièce murale par l'intermédiaire d'une articulation tournante, laquelle est prévue sur les deux faces frontales et orientée perpendiculairement à la plaque de fond (18).

9. Dispositif selon la revendication 7 ou 8, **caractérisé en ce que** la barre de butée (42), dans la première position de basculement, s'applique par les pieds (44) contre la face inférieure de la plaque de fond (18) et **en ce que** la barre de butée (42), dans la deuxième position de basculement, s'applique contre la première barre d'appui (22) et les pieds (44) reposent sur la surface de support (14).

10. Dispositif selon l'une quelconque des revendications précédentes, **caractérisé en ce que** les barres d'appui (22, 24) sont dimensionnées de manière identique.

11. Dispositif selon l'une quelconque des revendications précédentes, **caractérisé en ce que** des poignées (32) sont agencées sur les faces frontales (38).

12. Dispositif selon la revendication 11, **caractérisé en ce que** les poignées (32) sont agencées à une distance telle de la plaque de fond (18) qu'elles se situent au-dessus des barres d'appui (22, 24), en particulier à une distance au-dessus des barres d'appui (22, 24) qui est supérieure au rayon des évidements (26, 28) en forme de u, lesquels forment l'appui.

13. Dispositif selon l'une quelconque des revendications précédentes, **caractérisé en ce qu'**il est réalisé avec possibilité d'être autoclavé, en particulier **en ce qu'**il est réalisé en acier inoxydable.
